# EUROPÄISCHE PATENTSCHRIFT

(11) **EP 1 455 782 B1**
(45) Veröffentlichungstag und Bekanntmachung des Hinweises auf die Patenterteilung: **21.03.2007**
(21) Anmeldenummer: 02795137.5
(22) Anmeldetag: 10.12.2002
(51) Int. Cl.: A61K 31/427, A61K 31/5415, A61P 29/00, A61K 9/00

(54) **HOCHKONZENTRIERTE STABILE MELOXICAMLÖSUNGEN ZUR NADELLOSEN INJEKTION**
HIGH-CONCENTRATION STABLE MELOXICAM SOLUTIONS FOR NEEDLE-LESS INJECTION
SOLUTIONS STABLES DE MELOXICAM A FORTE CONCENTRATION POUR INJECTION SANS AIGUILLE

(30) Priorität: 12.12.2001 DE 10161077
(43) Veröffentlichungstag der Anmeldung: 15.09.2004
(73) Patentinhaber: BOEHRINGER INGELHEIM VETMEDICA GMBH, 55216 Ingelheim am Rhein (DE)
(72) Erfinder: HENKE, Stefan, 55239 Gau-Odernheim (DE); FOLGER, Martin, Andreas, 55218 Ingelheim (DE); HASSEL, Bernhard, 55437 Ockenheim (DE); ZIERENBERG, Bernd, 55411 Bingen (DE)
(86) Internationale Anmeldenummer: PCT/EP2002/013983
(87) Internationale Veröffentlichungsnummer: WO 2003/049733

(56) Entgegenhaltungen:
- US-B1- 6 284 269
- KISHORE KUMAR, N. ET AL.: "COMPARATIVE STUDIES ON EFFECT OF SOME HYDROPHILIC POLYMERS ON THE DISSOLUTION RATE OF A POORLY WATER SOLUBLE DRUG, MELOXICAM" INDIAN DRUGS, Bd. 39, Nr. 6, 19. April 2002 (2002-04-19), Seiten 323-329, XP008015692

## Beschreibung

Die vorliegende Erfindung betrifft, hochkonzentrierte stabile Meloxicamlösungen für die intrakutane oder subkutane nadellose Injektion zur Behandlung von respiratorischen Erkrankungen und Entzündungen bei Säugetieren.

### Hintergrund der Erfindung

Meloxicam (4-Hydroxy-2-methyl-N-(5-methyl-2-thiazolyl)-2H-1,2-benzothiazin-3-carboxamid-1,1-dioxid) ist ein zur Gruppe der NSAID's (non-steroidal-antiinflammatory drugs) gehörender Wirkstoff. Meloxicam sowie dessen Natrium- und Megluminsalz (N-Methyl-D-glucaminsalz) sind in EP-A-0 002 482 beschrieben. EP-A-0 002 482 zeigt u.a. das Beispiel einer 0,2 %igen injizierbaren Lösung des Meloxicams bestehend aus dem Megluminsalz des Wirkstoffes, Natriumchlorid und Wasser.
EP A-0 945 134 offenbart das pH-abhängige Löslichkeitsverhalten von Meloxicam und seinen Salzen, d.h. des Natriumsalzes, des Ammoniumsalzes und des Megluminsalzes, in wässriger Lösung. Danach ist Meloxicam ein in Wasser schwerlöslicher Wirkstoff. Die Meloxicamsalze, insbesondere das Megluminsalz, zeigen wie in Tabelle 1 aus EP 0 945 134 gezeigt eine verbesserte Löslichkeit mit steigendem pH-Wert zwischen 4 und 10.
Dennoch waren bisher nur stabile klare wässrige Lösungen mit einer niedrigen Meloxicamkonzentration herstellbar. Diese mußten neben der in situ Bildung eines Meloxicamsalzes z.B. Meglumin-Salzes und der Zugabe von Löslichkeitsvermittlern auch einen pH-Wert in dem Bereich der größtmöglichen Löslichkeit sowie akzeptablen Verträglichkeit aufweisen und einen hohen Anteil organischen Lösungsmittels enthalten. Formulierungsversuche mit gleicher oder ähnlicher Rezeptur führten bei höheren z.B. 2%igen Meloxicamkonzentrationen zu einer Trübung der Lösung.
W09959634 A1 beschreibt eine 0,5 % Meloxicam enthaltende Augentropfenlösung, gibt jedoch keinen Hinweis auf mögliche Meloxicamkonzentrationen über 1%.
Eine auf dem Markt erhältliche 0,5 %ige Meloxicamlösung wird bei kleinen Tieren wie Hunden, Jung-Rindern und Kälbern z.B. zur Behandlung respiratorischer Erkrankungen und Entzündungen angewendet. US-B-6 284 269 offenbart pharmazeutische Zubereitungen, die Meloxicam und Cyclodextrin enthalten.
Eine Wirkstofflösung zur nadellosen Injektion bietet dem Tierhalter die Möglichkeit dem Tier eine sterile Lösung selbst zu verabreichen. Zu den Anforderungen an eine Wirkstofflösung zur nadellosen Injektion gehören u.a. kleine injizierbare Volumina, eine gewichtsbezogene Dosierungsmöglichkeit sowie eine möglichst einstellige Anzahl von Auslösevorgängen pro Behandlungseinheit. Dementsprechend sind beispielsweise Injektionsvolumina von 50 µl pro Ausstoß technisch realisierbar. Dazu kann, wie in DE10010123 A1 beschrieben, eine sterile Lösung unter aseptischen Bedingungen in eine sterile Kartusche gefüllt werden, welche anschließend in das Dosiersystem eingesetzt wird .
Die Behandlung von Säugetieren mit einer nadellos injizierbaren Meloxicamlösung war bisher nicht möglich. Die niedrige Wirkstoffkonzentration der Injektionslösung ließ kein akzeptables, verträgliches Injektionsvolumen zu. Die Applikation von Meloxicamlösungen mittels nadelfreier Injektion erfordert eine Partikelfreiheit der Lösung, da an derartige Lösungen die gleichen Anforderungen wie an Lösungen zur parenteralen Applikation zu stellen sind. Neben intrakutaner und subkutaner Aufnahme ist auch eine transkutane Applikation zu berücksichtigen, was eine direkte Aufnahme in Blutgefäße bedeutet. Der letzte Weg ist direkt mit einer intravenösen Anwendung mittels Injektion durch eine Injektionsnadel zu vergleichen. Der Applikationsweg mittels nadelfreier Injektion hat einen relevanten Einfluß auf die Bioverfiigbarkeit, die Bioverfügbarkeit wird gegenüber intrakutaner oder subkutaner Applikation erhöht sein, da bei nadelfreier Injektion auch transkutane Resorption stattfindet. Organische Lösungsmittel, Lösungsvermittler und wasserlösliche Stoffe können aus Verträglichkeitsgründen nur in bestimmten Konzentrationen eingesetzt werden.
Es ist die Aufgabe der vorliegenden Erfindung langzeitstabile partikelfreie hochkonzentrierte Meloxicamlösungen herzustellen, welche zur nadellosen Injektion geeignet sind.

### Beschreibung der Erfindung

Überraschenderweise wurde gefunden, daß hochkonzentrierte Meloxicamlösungen mit einem Wirkstoffgehalt von 35 bis 100 mg/ml, die neben einem Meloxicamsalz sowie bestimmten Hilfsstoffen einen weiteren Hilfsstoff aus der Gruppe Citronensäure, Lecitin, Gluconsäure, Weinsäure, Phosphorsäure und EDTA oder deren Salze enthalten, partikelfrei und langzeitstabil herstellbar sind. Die Stabilität ließ sich mit einem unerwartet geringen Anteil organischer Lösungsvermittler erzielen. Auch gegenüber dem Verfahren einer Finalsterilisation konnte die Stabilität der Formulierung festgestellt werden.
Daraus ergibt sich die Aufgabenlösung der Erfindung als Formulierung einer Meloxicamlösung, die neben einem Meloxicamsalz, in geringen Konzentrationen Lösungsvermittler, einen Konservierungsstoff, eine Puffersubstanz zur Einstellung des optimalen pH-Bereichs und einen weiteren Hilfsstoff enthält.
Gegenstand der Erfindung sind wie in Anspruch 1 beschrieben wässrige intrakutan oder subkutan nadellos applizierbare cyclodextrinfreie Lösungen von Meloxicam, welche ein pharmakologisch verträgliches Meloxicamsalz einer organischen oder anorganischen Base in einer hochkonzentrierten Lösung mit 35-100 mg/ml Meloxicam zusammen mit geeigneten Hilfsstoffen enthalten. In den Unteransprüchen 2-14 werden vorteilhafte Weiterentwicklungen der Erfindung beschrieben.

Die erfindungsgemäße Formulierung überwindet das sich aus dem Stand der Technik ergebende Problem eine zur nadellosen Injektion geeignete Lösung des Wirkstoffs Meloxicam zu schaffen, indem sie mit einer im folgenden beschriebenen Zusammensetzung eine hohe Wirkstoffkonzentration in einer partikelfreien langzeitstabilen Lösung zuläßt.

Die erfindungsgemäße Formulierung kann als Meloxicamsalz das Meglumin-, Natrium-, Kalium oder Ammoniumsalz, bevorzugt das Meloxicam Megluminsalz enthalten.

Als Lösungsvermittler können beispielsweise Polyethylenglycole, Polyoxyethylen-Polyoxypropylen-Copolymere (z.B.Poloxamer 188), Glycofurol, Arginin, Lysin, Ricinusöl, Propylenglycol, Solketal, Polysorbat, Glycerol, Sorbitol, Mannitol, Xylitol Polyvinylpyrrolidone, Lecitin, Cholesterol, 12-Hydroxystearinsäure-PEG660-Ester, Propylenglycolmonostearat, Polyoxy-40-Hydriertes Ricinusöl, Polyoxyl-10-oleyl-ether, Polyoxyl-20-cetostearylether und Polyoxyl-40-stearat oder ein Sorbitol/Mannitol/Xylitol-Gemisch, vorzugsweise Polyethylenglycole, Polyoxyethylen-Polyoxypropylen-Copolymere , Glycofurol, Polyvinylpyrrolidone, Lecitin, Cholesterol, 12-Hydroxystearinsäure-PEG660-Ester, Propylenglycolmonostearat, Polyoxy-40-Hydriertes Ricinusöl, Polyoxyl-10-oleyl-ether, Polyoxyl-20-cetostearylether und Polyoxyl-40-stearat verwendet werden. Besonders bevorzugt sind Polyethylenglycole, Glycofurol und Polyoxyethylen-Polyoxypropylen-Copolymere, insbesondere jedoch Polyethylenglycole (z.B. Macrogol 300) und Polyoxyethylen-Polyoxypropylen-Copolymere (z.B. Poloxamer 188).
Als Konservierungsstoffe können beispielsweise Ethanol, Benzoesäure sowie deren Natrium-oder Kaliumsalze, Sorbinsäure, sowie deren Natrium- oder Kaliumsalze, Chlorbutanol, Benzylalkohol, Phenylethanol, Methyl-, Ethyl-, Propyl- oder Butyl-p-Hydroxybenzoesäureester, Phenol, m-Kresol, p-Chlor-m-Kresol oder Benzalkoniumchlorid enthalten sein. Besonders bevorzugt sind Ethanol, Benzoesäure sowie deren Natrium- oder Kaliumsalze, Sorbinsäure, sowie deren Natrium- oder Kaliumsalze, Chlorbutanol, Benzylalkohol, Phenylethanol und Methyl-, Ethyl-, Propyl- oder Butyl-p-Hydroxybenzoesäureester, vorzugsweise jedoch Ethanol, Benzoesäure sowie deren Natrium-oder Kaliumsalze, Sorbinsäure, sowie deren Natrium- oder Kaliumsalze, insbesondere jedoch Ethanol.
Als Puffersystem zur Einstellung eines pH-Werts zwischen 8 und 10 können beispielsweise Glycin, ein Gemisch Glycin / HCl, ein Gemisch Glycin/Natronlauge, sowie deren Natrium-und Kaliumsalze, ein Gemisch Kalium-hydrogenphthalat / Salzsäure, ein Gemisch Kaliumhydrogenphthalat / Natronlauge oder ein Gemisch Glutaminsäure / Glutamat eingesetzt werden. Besonders bevorzugt sind Glycin, ein Gemisch Glycin / HCl und ein Gemisch Glycin / Natronlauge, insbesondere Glycin.
Weitere geeignete Hilfsstoffe sind Citronensäure, Lecitin, Gluconsäure, Weinsäure, Phosphorsäure und EDTA oder deren Alkalisalze, bevorzugt Weinsäure und EDTA oder dessen Alkalisalze, insbesondere Dinatrium EDTA.
Eine Ausführungsform der Erfindung enthält neben dem Meglumin- oder Natriumsalz des Meloxicams, als Lösungsvermittler Polyethylenglycole, Glycofurol oder/und Polyoxyethylen-Polyoxypropylen-Copolymere, insbesondere jedoch Polyethylenglycole (z.B. Macrogol 300) oder/und Polyoxyethylen-Polyoxypropylen-Copolymere(z.B. Poloxamer 188), als KonservierungsstoffEthanol, Benzoesäure sowie deren Natrium- oder Kaliumsalze, oder Sorbinsäure, sowie deren Natrium- oder Kaliumsalze, insbesondere jedoch Ethanol und als Puffer Glycin, ein Gemisch Glycin / HCl oder ein Gemisch Glycin/Natronlauge, vorzugsweise jedoch Glycin und als weiteren Hilfsstoff Dinatrium EDTA.

Die erfindungsgemäße Formulierung kann Meloxicam in einer Konzentration von 35-100 mg/ml, vorzugsweise 40-80 mg/ml, bevorzugt 45-70 mg/ml, besonders bevorzugt 50-60 mg/ml, insbesondere etwa 55 mg/ml enthalten.
Die Megluminkonzentration kann zwischen 30 und 50 mg/ml, vorzugsweise 35-45 mg/ml, bevorzugt 38-42 mg/ml, besonders bevorzugt etwa 40 mg/ml betragen. Die möglichen Natrium-, Kalium- und Ammoniumkonzentrationen berechnen sich entsprechend.

Die Konzentration der Lösungsvermittler kann im Bereich von 20-200 mg/ml, vorzugsweise 30-150 mg/ml, bevorzugt 40-130 mg/ml, besonders bevorzugt 50-120 mg/ml, insbesondere 70-100 mg/ml liegen.
Die Konzentration des Konservierungsstoff Ethanol kann im Bereich von 100-200 mg/ml, vorzugsweise 120-180 mg/ml, besonders bevorzugt etwa 150 mg/ml liegen.
Die Konzentration der Konservierungsstoffe Benzoesäure sowie deren Natrium- oder Kaliumsalze, Sorbinsäure, sowie deren Natrium- oder Kaliumsalze, Chlorbutanol, Benzylalkohol, Phenylethanol, Phenol, m-Kresol und p-Chlor-m-Kresol kann im Bereich von 0,5-50 mg/ml, vorzugsweise 1-10 mg/ml, besonders bevorzugt 3-5 mg/ml liegen.
Die Konzentration der Konservierungsstoffe Benzalkoniumchlorid, Phenylquecksilbernitrat und Methyl-,Ethyl-,Propyl-oder Butyl- p-Hydroxybezoesäureester kann im Bereich von 0,01-4 mg/ml, vorzugsweise 0,02-3 mg/ml, besonders bevorzugt 0,1-0,5 mg/ml liegen.
Die Konzentration der Puffersubstanzen kann zwischen 4 und 138 mg/ml, vorzugsweise 5 und 20 mg/ml, bevorzugt 8 und 10 mg/ml betragen.
Die Konzentration der weiteren oben genannten Hilfsstoffe, d.h. EDTA, Citronensäure, Lecitin, Gluconsäure, Weinsäure und Phosphorsäure oder deren Salze kann im Bereich von 0,2-3 mg/ml, vorzugsweise 0,3-2,5 mg/ml, bevorzugt 0,5-2 mg/ml, besonders bevorzugt 0,6-1,5 mg/ml, insbesondere 0,7-1,0 mg/ml liegen.

Meglumin und Meloxicam können in einem molaren Verhältnis zwischen 9:8 und 12:8, vorzugsweise in einem molaren Verhältnis von 11:8, insbesondere jedoch in einem molaren Verhältnis von 10:8 eingesetzt werden.
In der erfindungsgemäßen Formulierung können Meloxicam und der weitere Hilfsstoff, insbesondere Dinatrium EDTA in einem Gewichtsverhältnis zwischen 100:1 und 35:1, vorzugsweise zwischen 80:1 und 40:1, bevorzugt zwischen 70:1 und 45:1, besonders bevorzugt zwischen 60:1 und 50:1, ganz besonders bevorzugt zwischen 58:1 und 52:1, insbesondere bei etwa 55:1 vorliegen.

Die erfindungsgemäße Formulierung kann eine Anbruchstabilität von 28 Tagen oder mehr aufweisen.
Die Lagerstabilität der Lösung bei geschlossener Originalverpackung kann bei einem Monat oder mehr, insbesondere zwischen 1 Monat und 24 Monaten, mindestens jedoch zwischen 1 Monat und 18 Monaten, bevorzugt zwischen 1 Monat und 12 Monaten, insbesondere bevorzugt zwischen 1 Monat und 9 Monaten, ganz besonders bevorzugt zwischen 1 Monat und 6 Monaten, insbesondere zwischen 1 Monat und 3 Monaten liegen.

Die erfindungsgemäße Formulierung sollte einen pH-Wert zwischen 8 und 10, bevorzugt zwischen 8,5 und 9, besonders bevorzugt eine pH-Wert zwischen 8,7 und 8,9, insbesonders von 8,8 aufweisen.

Die erfindungsgemäße Formulierung eignet sich zur Behandlung von Schmerz, Entzündungen, Fieber, akuter Mastitis, Diarrhoe, Lahmheiten, onkologischen Indikationen, Beeinträchtigungen des Bewegungsapparates und respiratorischen Erkrankungen bei Tieren, vorzugsweise von akuter Mastitis, Diarrhoe, Lahmheiten, Beeinträchtigungen des Bewegungsapparates und respiratorischen Erkrankungen, bevorzugt von akuter Mastitis, Diarrhoe, Lahmheiten, Beeinträchtigungen des Bewegungsapparates und respiratorischen Erkrankungen, insbesondere bevorzugt von respiratorischen Erkrankungen und onkologischen Indikationen. Die Behandlung kann in Verbindung mit einer antibiotischen Therapie erfolgen.

Die erfindungsgemäße Formulierung eignet sich zur Behandlung von Tieren, bevorzugt von Säugetieren, insbesondere Haustieren oder Nutztieren.

Die erfindungsgemäße Formulierung eignet sich zur Behandlung von Tieren, vorzugsweise von Tieren bis 500 kg, bevorzugt von Haustieren ab 1 kg, besonders bevorzugt von 2 bis 70 kg, insbesondere bevorzugt von 5 bis 60 kg, oder großen Tieren bis 750 kg, bevorzugt von 50 kg bis 500 kg, insbesondere bevorzugt von 100 bis 400 kg.

Die Dosierung der erfindungsgemäßen Formulierung sollte 0,1 bis 1,0 mg Wirkstoff/kg Körpergewicht, vorzugsweise 0,4 bis 0,8 mg/kg Köpergewicht, bevorzugt 0,5 bis 0,7 mg/kg Köpergewicht, insbesondere bevorzugt 0,6 mg/kg Köpergewicht entsprechen.

Die Herstellung der erfindungsgemäßen Formulierung kann nach aus der Literatur bekannten Herstellungsweisen für wässrige Flüssigformulierungen erfolgen. Es können beispielsweise zu einer Meloxicamsalzlösung die geeigneten Hilfstoffe hinzugefügt werden.

Als Verpackungsmaterial der erfindungsgemäßen Formulierung sind verschiedene handelsübliche Materialien für wässrige Flüssigformulierungen geeignet, die einen Verschluß unter Inertgas und/oder eine Finalsterilisation durch Autoklavieren im Endbehältnis ermöglichen. Dazu gehören z.B. Ampullen oder Glasvials, insbesondere Glasvials, beispielsweise 50 ml oder 100 ml Glasvials des Glastyps 1 (nach Pharm. Eur/ USP) in Kombination mit Gummistopfen aus Ethylenpropylennorbornen -terpolymer (EPDM) und Aluminiumkappen. Auch Vials aus Kunststoff, insbesondere aus COC (Cyclische Olefin-Copolymere), sowie andere Arten von Gummistopfen sind geeignet.

Die erfindungsgemäßen Meloxicamlösungen sollen durch das nachfolgende Beispiel erläutert werden. Dem Fachmann ist bewußt, daß dieses Beispiel nur zur Veranschaulichung dient und als nicht limitierend anzusehen ist.

### Beispiel

### 4%ige Meloxicamlösung

**Rezeptur:**

| | g/100 ml |
|---|---|
| Meloxicam | 4,0 |
| Meglumin | 2,8 |
| Macrogol 300*¹ | 15,0 |
| Poloxamer 188*² | 5,0 |
| Ethanol | 15,6 |
| Glycin | 0,5 |
| EDTA-Na | 0,1 |
| 1M HCl | q.s. ad pH 8,8 |
| 1M NaOH | q.s. ad pH 8,8 |
| Wasser für Injektion | ad 100 ml |

| | |
|---|---|
| *¹erhältlich von Brenntag, Plochingen, Deutschland *²erhältlich von C.H. Erbsloeh, Krefeld, Deutschland | |

Beispielsweise können Hunde mit einer erfindungsgemäßen 4%igen Meloxicamlösung bei einem Dosiervolumen von 50 µl pro Stoß mit einer genauen körpergewichtsbezogenen Dosierung mittels nadelfreier Injektion behandelt werden. Die Behandlung eines 10 kg schweren Hundes mit einer Dosis von 0,2 mg Meloxicam pro kg Körpergewicht ist mit genau einem Sprühstoß möglich. Eine Therapiegenauigkeit ist in diesem Fall in 10 kg Schritten gewährleistet.

### Herstellung:

4 g Meloxicam werden in 50 ml einer wässrigen Megluminlösung (1,4 g/50 ml) bei 90 °C gelöst. Die übrigen Hilfsstoffe werden gemäß obiger Rezeptur nacheinander der Lösung hinzugefügt. Anschließend wird ein pH-Wert von 8,8 mittels 1M Salzsäure und 1M Natriumhydroxid-lösung eingestellt. Die Lösung wird mit Wasser bis zur Erzielung des Volumens von 100 ml versetzt.

## Patentansprüche

1. Wässrige nadellos injizierbare cyclodextrinfreie Lösung von Meloxicam, enthaltend ein pharmakologisch verträgliches Meloxicamsalz einer organischen oder anorganischen Base, **dadurch gekennzeichnet, daß** sie einen Lösungsvermittler, sowie einen oder mehrere Hilfsstoffe ausgewählt aus der Gruppe bestehend aus Citronensäure, Lecitin, Gluconsäure, Weinsäure, Phosphorsäure und EDTA oder deren Salze enthält und der Gehalt an gelöstem Meloxicamsalz von 35 bis 100 mg/ml beträgt.

2. Wässrige Lösung nach Anspruch 1, **dadurch gekennzeichnet, daß** das Meloxicamsalz das Natrium- oder Megluminsalz ist.

3. Wässrige Lösung nach Anspruch 2, **dadurch gekennzeichnet, daß** sie Meglumin und Meloxicam in einem molaren Verhältnis zwischen 9:8 und 12:8 enthält.

4. Wässrige Lösung nach Anspruch 3, **dadurch gekennzeichnet, daß** sie Meglumin * und Meloxicam in einem molaren Verhältnis von 10:8 enthält.

5. Wässrige Lösung nach einem der Ansprüche 1- 4, **dadurch gekennzeichnet, daß** sie einen oder mehrere Hilfsstoffe aus der Gruppe der Puffer und/oder Konservierungsstoffe beinhaltet.

6. Wässrige Lösung nach einem der Ansprüche 1-5, **dadurch gekennzeichnet, daß** sie Di-Natrium EDTA enthält.

7. Wässrige Lösung nach einem der Ansprüche 1-6, **dadurch gekennzeichnet, daß** sie einen pH-Wert zwischen 8.0 und 10 aufweist.

8. Wässrige Lösung nach einem der Ansprüche 1-7, enthaltend Meloxicam, Meglumin, ein Polyethylenglycol, ein Polyoxyethylen-Polyoxypropylen-Copolymer, Ethanol, Glycin und gegebenenfalls Natriumhydroxid oder Salzsäure, **dadurch gekennzeichnet, daß** sie Di-Natrium EDTA enthält.

9. Wässrige Lösung nach einem der Ansprüche 1-7, im wesentlichen bestehend aus Meloxicam, Meglumin, ein Polyethylenglycol, ein Polyoxyethylen-Polyoxypropylen-Copolymer, Ethanol, Glycin, für die Injektion geeignetes Wasser und gegebenenfalls Natriumhydroxid oder Salzsäure, **dadurch gekennzeichnet, daß** sie Di-Natrium EDTA enthält.

10. Verwendung einer Lösung von Meloxicam, nach einem der Ansprüche 1-9 zur Herstellung eines Arzneimittels zur Behandlung von Schmerz, Entzündungen, Fieber und respiratorischen Erkrankungen bei Säugetieren.

11. Verwendung einer Lösung nach Anspruch 10, welche einem Dosierungsbereich von 0,1 bis 1,0 mg Wirkstoff /kg Körpergewicht entspricht.

12. Verwendung einer Lösung nach Anspruch einem der Ansprüche 10 bis 11 zur Herstellung eines nadellos injizierbaren Arzneimittels.

13. Sterile Kartusche enthaltend eine Lösung gemäß einem der Ansprüche 1 bis 9, welche in einem nadellosen Injektionssystem einsetzbar ist.

## Claims

1. Aqueous cyclodextrin-free solution of meloxicam suitable for administration by needleless injection, containing a pharmacologically acceptable meloxicam salt of an organic or inorganic base, **characterised in that** it contains a solubiliser, as well as one or more excipients selected from among the group comprising citric acid, lecithin, gluconic acid, tartaric acid, phosphoric acid and EDTA or the salts thereof, and the content of dissolved meloxicam salt is from 35 to 100 mg/ml.

2. Aqueous solution according to claim 1, **characterised in that** the meloxicam salt is the sodium or meglumine salt.

3. Aqueous solution according to claim 2, **characterised in that** it contains meglumine and meloxicam in a molar ratio of between 9:8 and 12:8.

4. Aqueous solution according to claim 3, **characterised in that** it contains meglumine* and meloxicam in a molar ratio of 10:8.

5. Aqueous solution according to one of claims 1 to 4, **characterised in that** it contains one or more excipients selected from among the buffers and/or preservatives.

6. Aqueous solution according to one of claims 1 to 5, **characterised in that** it contains disodium EDTA.

7. Aqueous solution according to one of claims 1 to 6, **characterised in that** it has a pH of between 8.0 and 10.

8. Aqueous solution according to one of claims 1 to 7, containing meloxicam, meglumine, a polyethyleneglycol, a polyoxyethylene-polyoxypropylene copolymer, ethanol, glycine and optionally sodium hydroxide or hydrochloric acid, **characterised in that** it contains disodium EDTA.

9. Aqueous solution according to one of claims 1 to 7, essentially consisting of meloxicam, meglumine, a polyethyleneglycol, a polyoxyethylene-polyoxypropylene copolymer, ethanol, glycine, water suitable for injection and optionally sodium hydroxide or hydrochloric acid, **characterised in that** it contains disodium EDTA.

10. Use of a solution of meloxicam according to one of claims 1 to 9 for preparing a pharmaceutical composition for treating pain, inflammation, fever and respiratory complaints in mammals.

11. Use of a solution according to claim 10, which corresponds to a dosage range of from 0.1 to 1.0 mg of active substance/kg of bodyweight.

12. Use of a solution according to one of claims 10 or 11 for preparing a pharmaceutical composition for needleless injection.

13. Sterile cartridge containing a solution according to one of claims 1 to 9, which can be used in a needleless injection system.

## Revendications

1. Solution aqueuse de méloxicam sans cyclodextrine injectable sans aiguille contenant un sel de méloxicam pharmacologiquement acceptable d'une base organique ou inorganique, **caractérisée en ce qu'**elle contient un promoteur de dissolution ainsi qu'un ou plusieurs adjuvants choisis dans le groupe consistant en l'acide citrique, la lécithine, l'acide gluconique, l'acide tartrique, l'acide phosphorique et l'EDTA ou leurs sels et la teneur en sel de méloxicam dissous est de 35 à 100 mg/ml.

2. Solution aqueuse selon la revendication 1 **caractérisée en ce que** le sel de méloxicam est le sel de sodium ou de méglumine.

3. Solution aqueuse selon la revendication 2 **caractérisée en ce qu'**elle contient de la méglumine et du méloxicam dans un rapport molaire entre 9 : 8 et 12:8.

4. Solution aqueuse selon la revendication 3 **caractérisée en ce qu'**elle contient de la méglumine et du méloxicam dans un rapport molaire de 10 : 8.

5. Solution aqueuse selon l'une des revendications 1-4 **caractérisée en ce qu'**elle contient un ou plusieurs adjuvants du groupe des tampons et/ou des conservateurs.

6. Solution aqueuse selon l'une des revendications 1 - 5 **caractérisée en ce qu'**elle contient de l'EDTA de disodium.

7. Solution aqueuse selon l'une des revendications 1-6 **caractérisée en ce qu'**elle présente un pH entre 8,0 et 10.

8. Solution aqueuse selon l'une des revendications 1 - 7 contenant du méloxicam, de la méglumine, un polyéthylèneglycol, un copolymère polyoxyéthylène-polyoxypropylène, de l'éthanol, de la glycine et éventuellement de l'hydroxyde de sodium ou de l'acide chlorhydrique, **caractérisée en ce qu'**elle contient de l'EDTA de disodium.

9. Solution aqueuse selon l'une des revendications 1 - 7 consistant essentiellement en méloxicam, méglumine, un polyéthylèneglycol, un copolymère polyoxyéthylène-polyoxypropylène, de l'éthanol, de la glycine, de l'eau appropriée pour l'injection et éventuellement de l'hydroxyde de sodium ou de l'acide chlorhydrique, **caractérisée en ce qu'**elle contient de l'EDTA de disodium.

10. Utilisation d'une solution de méloxicam selon l'une des revendications 1 - 9 pour la production d'un médicament pour le traitement de la douleur, des inflammations, de la fièvre et des maladies respiratoires chez les mammifères.

11. Utilisation d'une solution selon la revendication 10 qui correspond à un domaine posologique de 0,1 à 1,0 mg de principe actif/kg de poids corporel.

12. Utilisation d'une solution selon l'une des revendications 10 à 11 pour la production d'un médicament injectable sans aiguille.

13. Cartouche stérile contenant une solution selon l'une des revendications 1 à 9 qui peut être utilisée dans un système d'injection sans aiguille.
